# EUROPEAN PATENT SPECIFICATION

(11) **EP 4 593 890 B1**
(45) Date of publication and mention of the grant of the patent: **02.09.2026**
(21) Application number: 23810145.5
(22) Date of filing: 18.10.2023
(51) Int. Cl.: A61L 2/00, A61G 12/00, A61L 2/18, G16H 40/20, A61G 7/00

(54) **SYSTEM AND INTEGRATED METHOD OF STANDARDIZED AND AUTOMATED DISINFECTION IN HOSPITAL AND/OR HOME ENVIRONMENTS**
SYSTEM UND INTEGRIERTES VERFAHREN ZUR STANDARDISIERTEN UND AUTOMATISIERTEN DESINFEKTION IN KRANKENHAUS- UND/ODER HEIMUMGEBUNGEN
SYSTÈME ET PROCÉDÉ INTÉGRÉ DE DÉSINFECTION STANDARDISÉE ET AUTOMATISÉE DANS DES ENVIRONNEMENTS HOSPITALIERS ET/OU DOMESTIQUES

(30) Priority: 20.10.2022 IT 202200021645
(43) Date of publication of application: 06.08.2025
(73) Proprietor: P.R.I.S.M.E.D. SRL SOCIETÀ BENEFIT, 90143 Palermo (IT)
(72) Inventor: D'AMICO, Nicolo', 90144 Palermo (IT)
(74) Representative: Maroscia, Antonio
(86) International application number: PCT/IB2023/060525
(87) International publication number: WO 2024/084414

(56) References cited:
- CN-A- 105 709 249
- CN-A- 112 451 712
- US-A1- 2018 255 977
- US-A1- 2019 331 701

## Description

### Field of the invention

The present invention generally relates to the healthcare and patient care sector, and particularly relates to an integrated non therapeutic system and method for standardised and automated disinfection to promote the optimal treatment of patients in hospital and/or home environments.

### Background art

It is known that the increase of the average age of the population with chronic diseases is contributing to increasing the population of patients unable to independently take care of their hygiene in hospitals. These patients are mainly bedridden, they cannot take care of their personal hygiene independently and not all of them can be treated with the current sanitisation technologies.

Hospital infections are a bigger problem than healthcare. In Europe approximately 2.700.000 patients each year contract an infection during hospitalization (Cassini A, Plachouras D, Eckmanns T, Abu Sin M, Blank H-P, Ducomble T, et al. Burden of Six Healthcare-Associated Infections on European Population Health: Estimating Incidence-Based Disability-Adjusted Life Years through a Population Prevalence-Based Modelling Study. PLoS Med 2016;13(10): e1002150).

One aspect of great relevance is that many infections are caused by multi-resistant bacteria, resulting in increased morbidity and mortality. Furthermore, skin infections in long-term patients are a leading cause of bedsores, often causing systemic infections that can even lead to the death of the patient.

The social burden of these infections, as measured by a disability-adjusted life years index (DALYs), is 501 DALY per 100.000 inhabitants in the European Union (Mangen MJ, Plass D, Havelaar AH, Gibbons CL, Cassini A, Muhlberger N, et al. The pathogen- and incidence-based DALY approach: an appropriate [corrected] methodology for estimating the burden of infectious diseases.

Therefore, the proliferation of multi-resistant bacteria, fungi, and viruses on the skin of patients, the increase in hospitalisation due to complications from care-related infections, and in some cases even the increase in the mortality rate are some of the most important challenges faced by health services worldwide.

One of the most common ways of transmitting hospital infections is through contact with contaminated surfaces, including a patient's body surface. For this reason, several international guidelines for the prevention of hospital infections recommend hand hygiene of healthcare professionals with specific guidance on the hand washing procedure and hygiene of the patient's body ([1] Centers for Disease Control and Prevention. Guideline for hand hygiene in health-care settings. Recommendations of the healthcare infection control practices advisory committee and the HICPAC/SHEA/APIC/IDSA hand hygiene task force. MMWR Recomm Rep. 2002;51(RR-16):1-45; [2] WHO. Model List of Essential Medicines - Adults. 19th ed. World Health Organization; 2015:1-51http://www.who.int/medicines/publications/essential medicines/en/).

This is largely due to the absence of total, standardized, accurate and safe hygiene for bedridden patients. Infectious complications are an important cause of morbidity and mortality for attended patients, both in-patient and outpatient assisted, in hospitals, outpatient facilities and social care facilities. Such infections can adversely affect the outcome of the diagnosis and treatment processes and are responsible for significant additional costs for the national health system and the patient in question.

In Italy, it is estimated that 5% to 8% of hospitalised patients (450 - 700,000 people) have a hospital infection, mainly urinary infections, surgical wound infections, bedsores, pneumonia, and sepsis, and that for 4,500 - 7,000 subjects the infection is the leading or ancillary cause of death (Società Italiana Multidisciplinare per la Prevenzione delle Infezioni nelle Organizzazioni Sanitarie - Italian Multidisciplinary Society for the Prevention of infections in Health Care Organisations - (SIMPIOS). www.simpios.eu).

The economic impact on the healthcare system amounts to more than EUR 1.000.000,000 per year and the greatest burden lies in the prolonged hospitalisation.

In addition, 7,5% - 10% of hospitalization days are due to the onset of an infectious complication.

Nowadays, washing and sanitation of bedridden patients can be carried out using portable devices directly at the bed without the use of special bathtubs or showers.

Generally, these portable devices, used by the health care staff in the host or home facility, are of basin type with sponges, shower stretcher, bedside water delivery trolleys and the like.

One of the main disadvantages of these well-known washing and sanitation solutions for bedridden patients lies in that these portable devices generally have two separate reservoirs inside them, one for transporting clean water and one for collecting dirty washing water.

Since both tanks are placed inside the same portable device, there is a high risk of local contamination from viruses, germs and bacteria. This is due to the continuous increase in the pathogen load inside the tank containing dirty water, with the risk that viruses, germs and bacteria may not only leak into the surrounding environment, but also end up inside the tank of clean water to be dispensed to a patient.

A second disadvantage of such known portable devices is that the patient washing and sanitation service must be interrupted when clean water finishes and/or when the dirty water tank is full.

Furthermore, the portable device must be taken to the evacuation area or location, which further prolongs the treatment time.

A further disadvantage lies in the fact that there are no standardised, automated sanitation and decontamination treatments that allow for a complete and safe wash, providing data to improve the treatment and, where required, selecting the most suitable for the needs of a specific patient.

Therefore, the known methods for the sanitisation of bedridden patients are not safe and effective in preventing infections during the period of hospitalisation or bed rest.

Furthermore, not all bedridden patients are kept in hospital facilities, but some stay at home, therefore there arises the need for sanitation methods that can easily be implemented in private homes.

EP3646828 describes an integrated sterilization system and method with an ozone sterilisation cabinet, a robot for sterilising the floor and a system for controlling such devices conceived to eliminate or reduce the risk of infection or contamination in healthcare environments.

US2008/209665 describes a robotic device for sterilising floors adapted to sterilise hospital environments, while US8209051 discloses a remotely controlled robot for patient care. US2019331701 and CN112451712 teaches to use of robots and cobots for disinfecting a range of targets in health-care facilities. However, the above robots and cobots are not used for washing/disinfecting patients.
CN105709249 describes an ozone sterilization device and method for non-disposable items used in hospital operating rooms or wards, particularly larger equipment such as beds. The device uses a sterilization bag for receiving the item to be sterilized, an ozone generator fed by an oxygen supply line, and a suction line for creating negative pressure and removing residual ozone after treatment. CN105709249 concerns sterilization of hospital items, such as beds and bedside tables, rather than the standardized and automated disinfection of patients.

US2018/255977 describes a manual washing system for a newborn in an environment kept under sterile and controlled temperature conditions.

Microbiological controls and Microbiological Result Indicators can certainly be useful for evaluating and managing environmental sanitation processes, if and only if, they are carried out reliably, encoded in every step and standardised in every procedure. It is therefore appropriate to describe the sampling method in as much detail as possible and to standardise the procedures to be followed and the techniques to be used.

For hospitalised patients, Healthcare-Associated Infections (HAls) are among the most frequent complications that can occur in healthcare facilities as a result of healthcare programme, and they are one of the main problems in managing infectious risk. It is estimated that between 5% and 15% of patients admitted to hospital develop at least one HAI during their stay in the healthcare facility ([3] Allegranzi B, Bagheri Nejad S, Combescure C, Graafmans W, Attar H, Donaldson L, et al. Burden of endemic health-care-associated infection in developing countries: systematic review and metaanalysis. Lancet. 2011; 377(9761):228-41. Epub 2010/12/15. doi: S0140-6736(10)61458-4 [pii] doi: 10.1016/S0140-6736(10)61458-4 PMID: 21146207. [4] Cookson B, Mackenzie D, Kafatos G, Jans B, Latour K, Moro ML, et al. Development and assessment of national performance indicators for infection prevention and control and antimicrobial stewardship in European long-term care facilities. J Hosp Infect. 2013; 85(1):45-53. Epub 2013/08/13. doi: S0195- 6701(13)00194-1 [pii] doi: 10.1016/j.jhin.2013.04.019 PMID: 23932737. [5] Suetens C, Hopkins S, Kolman J, Diaz Ho gberg L. Point prevalence survey of healthcare associated infections and antimicrobial use in European acute care hospitals. Stockholm, Sweden: European Centre for Disease Prevention and Control. 2013).

### Technical problem

In the light of the background art, the technical problem addressed by the present invention is to provide a standardised disinfection method that reduces the risk of contamination in hospital and/or home environments.

### Summary of the invention

The object of the present invention is to solve the aforementioned problem by providing an integrated method for the standardised and automated disinfection of patients in hospital and/or home environments which is highly efficient and cost-effective.

Another object of the present invention is to provide a method of the type indicated above which allows to automate and improve the procedures for disinfecting a bedridden patient.

A further object of the present invention is to provide a method of the type indicated above which allows to select the best disinfection treatment suitable for a determined patient.

A particular object of the present invention is to provide a system for the standardised and automated disinfection of patients in hospital and/or home environments which facilitates the tasks of the healthcare operators guiding them during the treatment of a patient.

Another object of the present invention is to provide a system which reduces as much as possible the contamination from pathogens in hospitals and at home.

The objects mentioned above and others which will be more apparent hereinafter, are attained by an integrated non-therapeutic method for the standardised and automated disinfection of patients in hospital and/or home environments according to claim 1.

The method comprises the steps of providing at least one bed for each individual patient, identifying a non-independent patient to be washed and sanitised, providing at least one server having a storage unit, providing a plurality of standardised disinfection protocols with which there are associated digitalised instructions with ozonisation percentage, temperature and application time for washing and sanitising the patient and the storage thereof in the storage unit, and providing at least one collaborative robot and at least one autonomous robot connected to the at least one server and having control means configured to allow the autonomous mobility in hospital and/or home environments.

Subsequently, the method provides the steps of selecting from the storage unit a predetermined standardised disinfection protocol suitable for the identified patient and allocating to the at least one collaborative robot and to the autonomous robot the selected protocol and the position of the bed of the identified patient.

Then, there are provided the steps of collecting and decontaminating washing water through pre-established ozonisation so as to confer to the water suitable bactericidal/antiviral properties, and disinfecting the identified patient by distributing the decontaminated and ozonised washing water on the skin of the patient depending on the selected protocol.

Lastly, there are provided the steps of recovering the dirty water coming from the washing and disinfection of each patient and collecting in a special container the contaminated washing water to be conveyed to the draining system. According to the invention, the step of collecting and decontaminating the water for washing each identified patient is carried out autonomously by the collaborative robot by means of a quick coupling pipe connected to the water mains, an automatic ozone generation system, an ozone sensor, a first pump and a clean water tank.
Moreover, the step of disinfecting the identified patient is carried out autonomously by the collaborative robot by approaching the bed of the identified patient, collecting the decontaminated clean water from the tank using the first pump, conveying the clean water to a dispenser arranged at the end of a motor-driven articulated arm.
The steps of recovering and of collecting the dirty water are carried out autonomously by the collaborative robot and/or by the autonomous robot by suctioning the dirty water from a collection compartment positioned on the bed of the patient using a second pump and pouring thereof into the specific container.

According to the invention, the collaborative robot is configured to collect data relating to the treatment of each individual patient and the data are transmitted by the collaborative robot to a storage unit connected to at least one central server and they are processed to make them available to the healthcare operators.

This will allow to analyse the collected data, compare them with standard parameters and parameters known in literature so as to monitor the quality of the treatments carried out on the patients and improve them.
Furthermore, a system is provided for carrying out the standardised and automated disinfection of patients in hospital and/or home environments comprising at least one bed for each individual patient, at least one central server with a storage unit on which there are stored disinfection protocols which are standardised and digitalised in the form of smart contracts, at least one collaborative robot, at least one autonomous robot, at least one medical kit and at least one buffer for collecting and distributing medical kits, wherein the robots comprises means for collecting and transmitting data relating to the treatment of the identified patient, collecting and decontaminating washing water, disinfecting the identified patient by distributing decontaminated and ozonised washing water on the skin of the patient, recovering the dirty water coming from the washing and disinfection of each patient.

Furthermore, the system comprises means for recovering and collecting in special containers the dirty water coming from the washing and disinfection of each patient to be conveyed to the draining system and the at least one collaborative robot comprises a tank for clean water, a first pump for dispensing clean water, an articulated arm provided with dispenser, an ozone generation system, an ozone sensor, a thermostat and a second pump for suctioning dirty water.

Advantageously, the at least one autonomous robot, the at least one central server and the at least one buffer are operatively connected and integrated to allow the controlled implementation of the standardised disinfection protocols as well as the exchange and storage of data and information relating to disinfection treatments for the healthcare management and for one or more healthcare operators.
Moreover, the at least one server is configured to monitor and process the data and the information, the condition of the beds for bedridden patients, of the medical kits, of the at least one collaborative robot and of the at least one autonomous robot through internet of things.

Thanks to these features, the system allows the standardised and automated treatments for each individual patient, making changes where necessary depending on the analysis of the collected data.

Hereinafter, the expression "collaborative robot" is used to indicate a robot configured to dispense in an automated fashion the clean and decontaminated water, in a determined amount, at a determined temperature with a determined sanitising property, over a predetermined period of time, on the specific areas of the body of the patient, capable of monitoring at least one healthcare operator during the sanitising treatment on the patient based on a smart contract, guiding the healthcare operator step-by-step along a predetermined programme, identifying the bed, the designated operator, the buffer, the kit and an autonomous robot.

Hereinafter, the expression "autonomous robot" will be used to indicate a robot configured to move bags of clean water, clean medical kits, used medical kits and bags containing dirty water, identifying the bed, the designated operator, the buffer of the ward, the kit and the collaborative robot.

Hereinafter, the expression "buffer" or "local buffer" will be used to indicate an automated dispenser located in a ward or at home, connected to the Internet and adapted to operate as a mini-storage unit, provided with sensors for reading RFID tags, QRcodes, barcodes provided to update the warehouse stock through automated loading and unloading functions. The buffer may identify a collaborative robot, one or more healthcare operators, the medical kits and one or more autonomous robots to whom there can be delivered one or more kits autonomously for example by dropping.

To this end, the autonomous robot may be configured to be suitably positioned with respect to the buffer so as to allow easy transfer of the number of kits indicated for each working day to deliver them to the individual beds even using the collaborative robot.

### Brief description of the drawings

Further features and advantages of the invention will be more apparent in the light of the detailed description of a preferred but not exclusive embodiment of an integrated method and system for the standardised and automated disinfection of bedridden patients with reference to the drawings below, wherein:
**FIG. 1** is a block diagram of the steps of the method for the sanitisation and decontamination of patients according to the invention;
**FIG. 2** is a partial perspective representation of the system for implementing the method according to the invention;
**FIG. 3** is a perspective view of a bed for a patient to be treated according to the method of Fig. 1;
**FIG. 4** is a cross-sectional lateral view of a collaborative robot according to the invention;
**FIG. 5** is a perspective view of the collaborative robot of Fig. 4, in an operative step;
**FIG. 6** is a cross-sectional lateral view of an autonomous robot according to the invention;
**FIG. 7** is a perspective view of a buffer according to the invention;
**FIG. 8** is a perspective view of flexible sheet for a bed of a patient which is not part of the invention but improves the understanding of the same;
**FIG. 9** is a perspective view of the flexible sheet of Fig. 8 hooked to a bed of Fig. 3;
**FIG. 10** is a perspective view of an alternative embodiment of a flexible sheet which is not part of the invention but improves the understanding of the same;
**FIG. 11** is a partial perspective representation of the step for washing and sanitising a patient according to the invention;
**FIG. 12** is a schematic representation of the components and functions of a central server according to the invention;
**FIG. 13** is a perspective view of a composting station according to the invention which is not part of the invention but improves the understanding of the same.

### Detailed description of a preferred embodiment

With particular reference to the figures, a method and a system for the standardised and automated disinfection of bedridden patients are described which are adapted to reduce as much as possible the risk of infection by pathogenic agents in the ward or at home.

Generally, the method and the system are applied in hospital and/or home environments where one or more beds are present for accommodating bedridden patients who are not able to independently take care of their personal care effectively.

Hereinafter, the expression "disinfection" will be used to indicate a treatment for washing and cleaning the skin of a patient adapted to remove and inactivate microorganisms, such as for example bacteria, viruses and fungi.

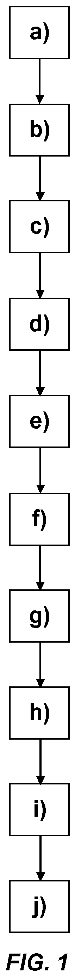

As shown in the block diagram in **FIG. 1****,** the integrated method for standardised and automated disinfection of patients comprising the following steps:
**a)** providing at least one bed **2** for each individual patient **P;**
**b)** identifying a non-independent patient **P** to be washed and sanitised;
**c)** providing at least one central server **7** having a storage unit;
**d)** providing a plurality of standardised disinfection protocols with which there are associated digitalised instructions with ozonisation percentage, temperature and application time for washing and sanitising the patient **P** and storing thereof on the storage unit;
**e)** providing at least one collaborative robot **3** and at least one autonomous robot **4** connected to the at least one server **7** and having control means configured to allow the autonomous mobility thereof in the hospital and/or home environments;
**f)** selecting from the storage unit a predetermined standardised disinfection protocol suitable for the identified patient **P** and allocating to at least one collaborative robot **3** and to the autonomous robot **4** the selected protocol and the position of the bed of the identified patient **P;**
**g)** collecting and decontaminating washing water through the pre-established ozonisation so as to confer to the water suitable bactericidal/antiviral properties;
**h)** disinfecting the identified patient **P** by distributing decontaminated and ozonised washing water on the skin of the patient **P** depending on the selected protocol;
**i)** recovering the dirty water coming from the washing and disinfection of each patient **P;**
**j)** collecting in a special container **5** the contaminated washing water to be conveyed to the draining system.

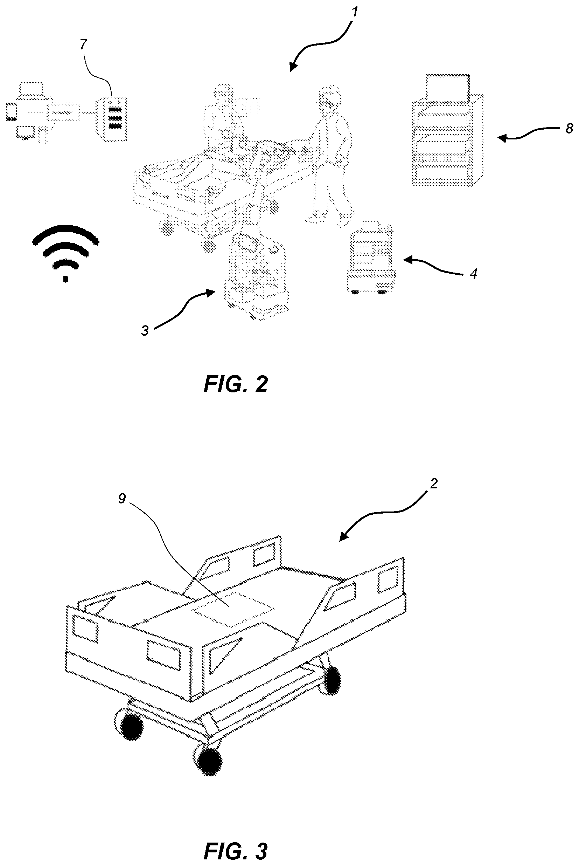

The standardised and automated disinfection method is implemented by a system, shown in its entirety in **FIG. 2** with the reference numeral **1,** which comprises:
- at least one bed **2** for a bedridden patient **P;**
- at least one central server **7** with a storage unit on which there are stored disinfection protocols which are standardised and digitalised in the form of smart contracts;
- at least one collaborative robot **3;**
- at least one autonomous robot **4;**
- at least one medical sanitisation and protection medical kit **6;**
- least one buffer **8** for collecting and distributing medical kits **6;**
- means for recovering and collecting in special containers **5** the dirty water coming from the washing and disinfection of each patient **P** to be conveyed to the draining system.

Hereinafter, the expression "at least one" or "a" will be used to indicate that in the hospital facility or at home there is present at least one of the entities described, without any numerical limit.

Each bed **2** for each patient **P** is provided with a sensor for detecting the presence of the patient **P** and the mobility ability thereof, and a unique RFID tag.

Obviously, the RFID tags used in the present invention may be replaced by similar means, such as for example QR code, NFC, barcode and the like

To this end, as better visible in **FIG. 3****,** the central body of each bed **2,** adapted to accommodate a patient **P,** has a sensor **9** which is a pressure sensor configured to detect the presence of the patient **P** continuously or at predetermined regular time intervals.

The pressure sensor **9** is capable of detecting not only the presence of the patient **P** in the bed but also the distribution of the pressure exerted on the surface of the bed when the patient moves, hence and it is used to indirectly detect the degree of mobility of the patient **P.**

The data relating to the presence and the pressure of a patient **P** in a bed **2** are sent from the pressure sensor **9** to the central server **7.**

The central server **7** stores, in the storage unit thereof, the data coming from the various pressure sensors **9** of the plurality of beds **2** and processes them to monitor which beds **2** are currently occupied and the availability of vacant beds **2.**

If the central server **7** does not receive data from a given bed **2** means that, at the time, the patient **P** is not present or that the bed thereof is vacant. If, instead, it receives data spaced out over time, this means that the patient **P** has a given degree of mobility given that the patient can get up from the bed **2** occasionally. Lastly, a constant or almost constant value will indicate a patient **P** with poor or no mobility at all, and therefore unable to get up tom the bed **2.**

Therefore, such configuration can monitor the hospitalisation of each individual patient **P** and identifying the non-independent patient/s **P** to be washed and treated according to step **b)** of the present method.

Furthermore, the continuous monitoring will allow to suitably plan when and to which patients **P** to send the care of the healthcare operators **O** to wash and sanitise them.

Preferably, the clean water to be used to wash a patient **P** is collected autonomously by a collaborative robot **3.**

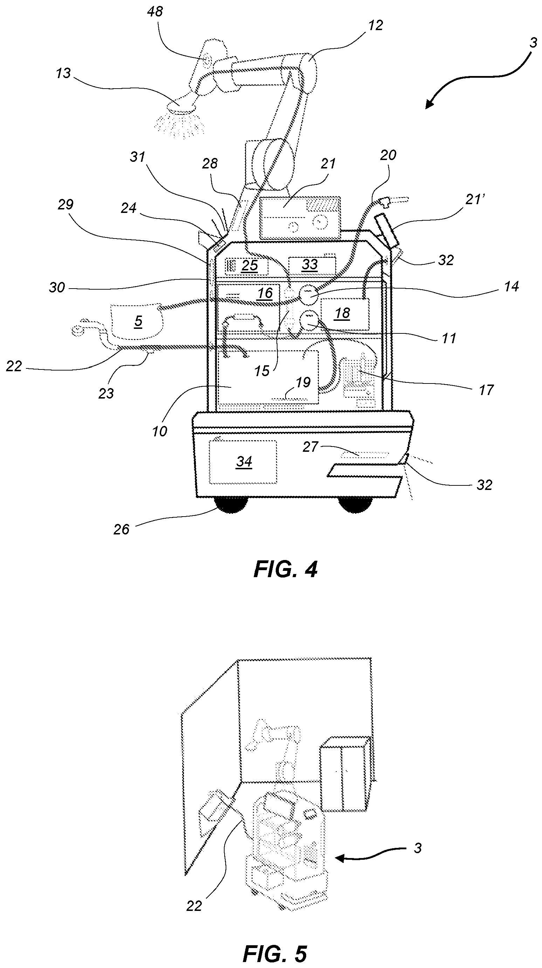

In a preferred embodiment, shown in **FIG. 4****,** the collaborative robot **3** comprises a tank **10** for clean water, a first pump **11** for dispensing clean water, an articulated arm **12** with dispenser **13** for clean water, a second pump **14** for suctioning dirty water, a flow meter **15,** an ozone generation system **16,** an ozone sensor **17,** an ozone dispensing system **18,** a thermostat **19,** a disinfecting system **20** and a pair of touchscreen monitors **21, 21'.**

In a per se known manner, the clean water may be decontaminated by replacing the ozone generation system **16** with similar components, such as for example UV lamps, chlorhexidine dispenser, and other systems known to a person skilled in the art.

As shown in **FIG. 5****,** using a quick coupling pipe **22,** the collaborative robot **3** may be autonomously connected to the water mains so as to fill the clean water tank **10.** The pipe **22** may be provided with an anti-legionella micro-filter **23** for filtering the water from the water mains.

The water is suctioned into the tank **10** by the first pump **11,** which then, during the automated disinfection step **h),** will convey water from the tank **10** to the articulated arm **12** where it will be dispensed through a dispenser **13.**

Advantageously, the clean water tank **10** may be provided with a filling valve adapted to shut off the water supply when the tank **10** is full.

Furthermore, the tank **10** is connected to the ozone generation system **16,** which produces a determined amounts of ozone which is mixed with the clean water of the tank **10** through a Venturi valve **24.**

By so doing, the collaborative robot **3** carries out the step **g)** of collecting and decontaminating water in an automated manner removing potential bacteria and viruses and conferring to the water a determined level of bactericidal/antiviral properties.

Suitably, the ozone generation system **16** produces an amount of ozone which depends on the type of washing intended to be carried out, the needs of the patient **P** undergoing treatment, and the amount of clean water present in the tank **10** measured using a float, not shown in the drawings.

In a per se known manner, the temperature of the water in the tank is regulated by the thermostat **19** and controlled through an incorporated thermometer.

The ozone sensor **17** measures the amount of ozone introduced into the clean water to monitor the concentration thereof and maintain it within the desired range.

Advantageously, monitoring the ozone concentration through the sensor **17** allows to change the production of ozone and the introduction thereof into the tank **10** depending on the volume, the temperature and the concentration in the water already present in the tank **10.**

The ozone dispensing system **18** is adapted to dispense in the air the produced ozone for the external surface self-disinfection when the robot is found in a hermetically sealed room.

When the clean water tank **10** is sufficiently full, the collaborative robot **3** is capable of autonomously reaching the bed **2** on which the disinfection method will be carried out autonomously.

To this end, the collaborative robot **3** further comprises a control unit **25,** drive means **26,** a plurality of movement and geolocation sensors **27,** an RFID reader **28,** a microphone **29,** an audio system **30,** LED lights **31,** a plurality of video cameras **32,** and self-diagnosis means **33.**

Thanks to the drive means **26,** such as for example wheels driven by an electric motor (not shown in the figures), the collaborative robot **3** is capable of moving autonomously in hospital and home environments up to reaching the selected bed **2.**

Suitably, the collaborative robot **3** is provided with video cameras **32** adapted to detect potential obstacles and/or people along the path so as to avoid them.

The precise position of the bed **2** is sent from the central server **7** to the control unit **25** of the collaborative robot **3.**

In a per se known manner, the various electronic components of the collaborative robot **3** are supplied by a rechargeable battery **34.**

When the battery **34** is detected to be too low, the collaborative robot **3** will autonomously proceed to one of the charging stations, not shown in the figures.

The collaborative robot **3** may move to one of these charging stations even when one of the self-diagnosis means **33** detects any kind of malfunction (e.g.: excessive vibrations in a component, high temperature, etc.), sending a signal to the central server **7** so that an operator connected to the internal network of the hospital or remotely connected to home care can view an alert message using a computer, tablet or similar device.

Of course, the collaborative robot **3** may be provided with electrical outlets and extension cords so that it can be connected by means of electrical conductors, not shown in the figures, to a mains outlet so as to allow the use of the current without consuming its battery **34** if necessary.

This arrangement is particularly advantageous during the step **h)** of the automated disinfection of a patient **P** given that the collaborative robot **3** may be connected to a power outlet close to the bed **2** for the entire duration of the treatment without the risk of running out of power.

Advantageously, during the disinfection step **h),** or in any similar situation of need, should the clean water in the tank **10** be insufficient or almost finished, the autonomous robot **4** may refill it.

As a matter of fact, there may be provided bags containing clean water which may be collected by autonomous robot **4** and taken to the collaborative robot **3** so as to refill the tank **10** through the quick coupling pipe **22** described above.

Therefore, the step for the automated supply of clean water is carried out by an autonomous **4** and/or collaborative **3** robot.

If necessary, also the collaborative robot **3** will be capable of moving towards to the clean water bags and fill its tank **10** autonomously.

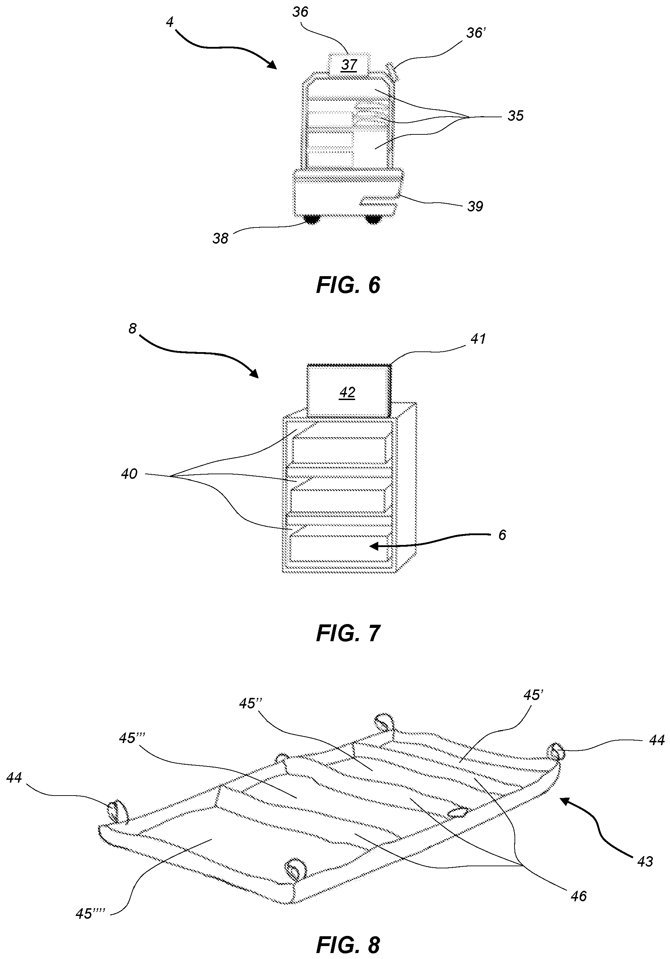

As visible in **FIG. 6****,** the autonomous robot **4** comprises a plurality of internal compartments **35,** a pair of touchscreen monitors **36, 36',** an RFID reader **37,** a control unit (not shown in the figures), drive means **38** and a plurality of movement and geolocation sensors **39.**

Similarly to the collaborative robot **3,** also the autonomous robot **4** will be provided with a rechargeable battery for the durable operating time thereof, and it may autonomously move in hospital wards and at home. Furthermore, the autonomous robot **4** may also be provided with a microphone and with an audio system (not shown in the figures).

Hereinafter will be described in detail, by way of non-limiting example, a preferred process for the standardised and automated disinfection of a patient **P** according to the invention.

Initially, the hospital facility, or the home care facility, establishes standard washing procedures, hereinafter referred to as disinfection protocols, and how to dispense them selecting from the procedures already known in the state of the art, by digitalising such instructions into computer codes.

Each protocol will contain information relating to the sequence of the areas of the body to be treated, the sequence of the medical devices to be used, the number of washings on the patient, the cleaning of the hands of the operators, and the amount of products, water and other parameters which need to be complied with so as to correctly carry out the given disinfection procedure.

To this end, each digitalised standardised disinfection protocol is encoded in a respective smart contract stored in the storage unit of the central server **7.**

As known, smart contracts are a set of computer instructions which promote, verify, or enforce compliance with the precise performance of a predetermined activity.

Therefore, each smart contract entered contains the specific information to be performed during the steps **g)** for the automated disinfection of the washing water, and **h)** for the automated disinfection of the skin of each individual patient **P.**

Furthermore, the smart contracts contain the information useful to the collaborative **3** and autonomous robots **4** so as to move in hospital and/or home environments in which the method of the present invention is carried out.

The use of smart contracts will limit the risks of erroneous washing and disinfection treatments given that the instructions contained therein must be strictly complied with.

Suitably, all entered smart contracts are stored in the central server **7.**

Subsequently, the pressure sensors **9** present in each individual bed **2** will allow to carry out the step **b)** of identifying a non-independent patient **P** who does not need to be washed and sanitised.

Besides the smart contracts, the central server **7** will also contain medical information of each individual patient **P,** therefore allowing the designated staff to select the standard protocol (and therefore the smart contract) most suitable for the needs of the given patient **P.**

Upon selecting the type of standard protocol, the information contained in the specific smart contract and the information relating to the position of the given patient **P** are sent from the central server **7** to at least one collaborative robot **3** and to at least one autonomous robot **4.**

Should the selected collaborative robot **3** need clean water, it will be supplied as described above, otherwise it will autonomously move towards the designated bed **2.**

Similarly, even some healthcare operators **O** will be warned and instructed to go to the selected bed **2** for carrying out activities for supporting the collaborative robot **3** during the automated disinfection step **h)** of distributing washing water on the skin of the patient **P.**

For example, the healthcare operators **O** may receive instructions from the central server **7** to go to a determined bed **2** on a personal portable electronic device thereof of the known type, such as for example a pager, a mobile phone, a tablet or the like.

Also the autonomous robot **4** will receive instructions relating to the position of the bed **2.**

Therefore, the information contained in each specific smart contract are sent from the central server **7** to at least one collaborative robot **3** and/or at least one autonomous robot **4** to autonomously carry out steps **g)** to **j).**

Furthermore, the autonomous robot **4** will receive the consent of going to a buffer **8** where to collect at least one sanitisation and protection medical kit **6** to be used when washing the patient **P.**

As a matter of fact, each standard protocol provides for the use of medical kits **6** for carrying out the step **h)** of disinfecting patients **P** under safe health conditions.

Suitably, each hospital ward or home is provided with at least one buffer **8** for the safe collection and controlled distribution of the medical kits **6** to wash the patients **P.**

As better shown in **FIG.7****,** each buffer **8** comprises a plurality of internal compartments **40,** for the safe collection and controlled distribution of medical kits **6,** a touchscreen monitor **41,** an RFID reader **42,** a control unit and a recognition system (not shown in the figures) adapted to interact with the operators **O** and the autonomous **4** and collaborative **3** robots.

Therefore, each buffer **8** is capable of recognising and granting access to the internal compartments **40** thereof to an operator **O,** or an autonomous robot **4** and a collaborative robot **3,** recording who, when, where he/she collected or loaded the medical kits **6** and the amount thereof.

Obviously, besides the medical kits **6,** each buffer **8** may also contain other objects useful for the tasks of the hospital facility.

All this information will be sent to the central server **7.**

Furthermore, keeping track of the number of medical kits **6** contained therein, each buffer **8** may send a request to the central server **7** to order the loading of new kits **6** if the amount is insufficient.

Advantageously, each medical kit **6** comprises disposable or reusable medical devices and a flexible laminar sheet **43.**

By way of non-limiting example, the medical devices contained in each kit **6** may be gowns or other personal protective equipment for the operators **O,** washing mittens/sponges, detergents, pads for drying the skin of the patient **P,** disinfectants, etc.

Furthermore, associated with each medical kit **6** is a unique RFID tag. Thanks to such RFID tag, each kit **6** may be identified individually, knowing the exact content, tracing it during the entire path thereof within the hospital facility or at home where the present method is carried out.

Obviously, instead of an RFID tag, each kit **6** may be provided with a QRcode, or a barcode, or the like.

After starting these initial steps, at least one collaborative robot **3** and at least one autonomous robot **4** will be at the bed **2** indicated by the central server **7** to carry out the automated disinfection of step **h).**

Each healthcare operator **O** sent to the bed **2** is identified by the present collaborative robot **3,** for example by reading the fingerprints by touching the touchscreen monitor **21, 21',** or through facial recognition carried out by the video cameras **32.**

Depending on the instructions contained in the given smart contract, the collaborative robot **3** timely starts the decontamination of the clean water producing a given amount of ozone to be mixed with the water, so as to carry out the steps **g)** of the present method.

By way of non-limiting example, if the smart contract indicates a washing treatment for the legs of the patient **P,** the ozone concentration in the clean water may be comprised between 1.20 and 1.40 mg/L.

Obviously, even the amount of the dispensed clean and decontaminated water and the temperature thereof will be predetermined depending on the type of selected washing, so as to be appropriate and at the same time comfortable for the patient **P.**

The healthcare operators **O** may therefore access, through the touchscreen monitor **21, 21'** of the collaborative robot **3,** the disinfection treatment details, by carefully reading the instructions to be carried out on the patient **P** and being guided in the support activity by the collaborative robot **3.**

Suitably, the patient **P** will be arranged on the flexible laminar sheet **43** present in the medical kit **6** provided by the autonomous robot **4.**

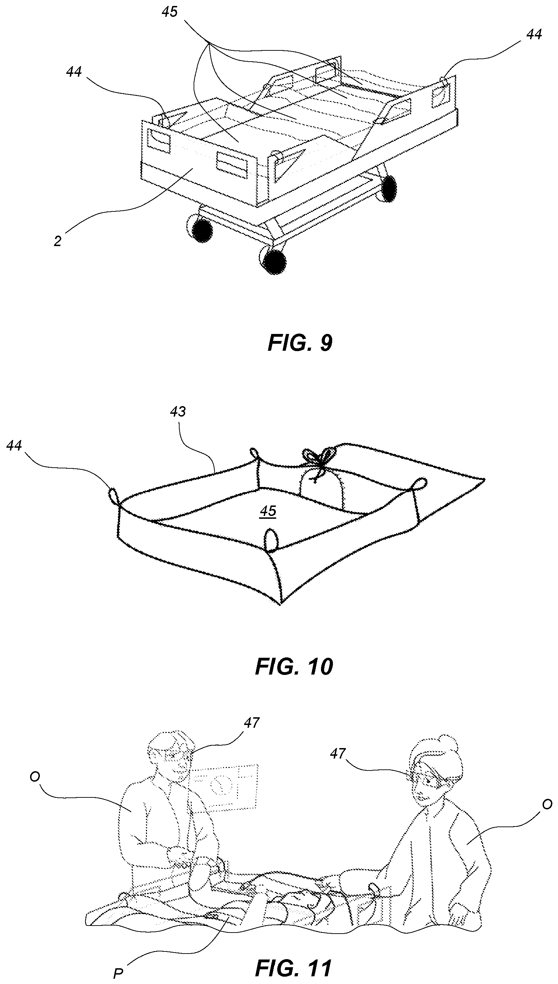

As visible in **FIGS. 8** and **9****,** the flexible laminar sheet **43** is provided with hooking means **44** for the bed **2** and with a plurality of compartments **45** intended to house the patient **P.**

Preferably, there are four compartments **45** and they are separated from each other by three partitioning members **46** perpendicular to the support surface of the sheet **43.** A compartment **45'** is designed to accommodate the head of the patient, one for the chest **45",** one for the private parts **45‴** and the other for accommodating the legs **45ʺʺ.**

The support surface and the partitioning members **46** are made of non-woven fabric that is recyclable and easy to disinfect, sterilise or transfer.

In a preferred embodiment, the hooking means **44** are webs made of non-woven fabric, or other appropriate fabrics, which pass through the partitioning members **46** so as to configure a given rigidity thereto.

**FIG. 10** shows an alternative embodiment of a partial flexible sheet **43** which has a single compartment **45** configured to house a given part of the body of the patient **P.**

In particular, the sheet **43** of **FIG. 10** has a compartment **45** adapted to house and contain the private parts of the patient **P.** Therefore, there will be provided for sheets **43** with compartments **45** of various shapes adapted to house the various individual parts of a patient P.

Furthermore, there may be provided for laminar sheets with different shapes, for example configured to house only the face, the legs or other parts of the human body.

After hooking the flexible sheet **43** to the bed **2,** or other appropriate support object, the healthcare operators **O** may house the patient **P** in the sheet **43** resting on the various parts of the body between the compartments **45** as described above.

Subsequently, the healthcare operators **O** may start the activities scheduled during the automated dispensing of the washing water in the area, or in the areas, indicated in the protocol contained in the reference smart contract, therefore supporting the collaborative robot **3** during the disinfection step **g).**

As described above, the collaborative robot **3** will reach the bed **2** indicated with the tank **10** thereof filled with water at a given temperature and with a given ozone concentration as indicated in the implemented protocol.

Once the patient **P** has been prepared by the healthcare operators **O,** the collaborative robot **3** will start dispensing the decontaminated water in the area of the indicated patient.

Also the amount of decontaminated water and the dispensing time are parameters contained in the implemented smart contract.

Suitably, through the dispenser **13** of the articulated arm **12,** the collaborative robot **3** may proceed with the automated dispensing of the decontaminated and clean water on the correct area of the skin of the patient **P,** guiding the support activities of the healthcare operators **O** and also to the use of the detergents contained in the medical kit **6** as defined in the disinfection protocol of the specific smart contract.

Therefore, the step **h)** of disinfecting the identified patient **P** is autonomously carried out by the collaborative robot **3** by approaching the bed **2** of the identified patient **P,** collecting the decontaminated clean water from the tank **10** using the first pump **11,** and conveying the clean water to the dispenser **13** arranged at the end of the motor-driven articulated arm **12.**

Advantageously, the healthcare operators **O** may continuously check the steps carried out as well as those to be carried out, as well as all the parameters directly in the touchscreen monitor **21, 21'** of the collaborative robot **3.**

Given that it is connected to the central server **7,** the collaborative robot **3** may provide to the operators **O** the information for supporting the performance of each selected protocol besides medical information stored in the server **7,** or which can be found online, to help the healthcare operators **O** in their work.

Further, each healthcare operator **O** may be provided with smart-glasses **47,** shown in **FIG. 11****,** connected to the collaborative robot **3** and/or to the central server **7.**

The smart-glasses **47** are provided with lenses on which there is integrated a micro-screen adapted to project the same information available on the touchscreen monitor **21, 21'** of the collaborative robot **3** or coming from the server **7.**

In this manner, the healthcare operators **O** may continuously carry out the support activities provided for without looking away from the patient **P** to verify given information relating to the ongoing treatment.

Furthermore, the smart-glasses **47** may detect any dirt by analysing the skin of the patient **P.**

This characteristic is significantly advantageous in the case where the healthcare operators **O** are designated to support the collaborative robot **3** when disinfecting the skin of the patient **P** should there be a lesion.

As a matter of fact, especially in the case of bedsores, it is very important for the operator **O** to focus on the point of treatment so as to avoid potential errors which could worsen the conditions of the patient **P.**

Therefore, the screens integrated in the lenses of the smart-glasses **47,** allow the healthcare operators **O** to simultaneously monitor the point featuring the lesion and the information sent by the collaborative robot **3** without having to view the touchscreen monitor **21, 21'** looking away from the patient **P.**

Furthermore, through the smart-glasses **47,** the collaborative robot **3** may display the lesion attributing it a stage and size, so as to possibly select a protocol indicated by the structure for pressure ulcers with similar characteristics (size, stage, etc.).

Furthermore, using the smart-glasses **47,** the healthcare operators **O** may photograph the ulcer (or the other present lesions) day by day through a simple voice command.

The images thus acquired are sent to the central server **7** so as to be stored and they may be subsequently analysed or used as comparison with others so as to follow and evaluate the condition of the lesion/s so as to implement the best procedures possible.

Another advantage lies in the fact that in the control unit **25** of the collaborative robot **3** there is installed an artificial intelligence software that enables the dialogue with the healthcare operators **O** in real time.

Therefore, the healthcare operators **O** may interact with the collaborative robot 3 through queries and/or voice commands.

The dirty washing water, which is accumulated in a given compartment **45,** is suctioned by the second pump **14** of the collaborative robot **3** and moved away towards a container or bag **5** provided near the bed **2,** therefore carrying out the steps **h)** of the recovery and **i)** of automated collection of the dirty water according to the present method.

In this manner, the dirty water will be rapidly moved away from the patient **P.** Furthermore, it will not be stored near the clean water tank **10,** avoiding any contaminations.

Therefore, the steps **h)** of the disinfection, **i)** of recovery e **j)** and of automated collection of the dirty water are carried out by a collaborative robot **3,** possibly supported by the healthcare operators **O.**

At the end of the treatment, or when the container **5** is full, the autonomous robot **4** or a healthcare operator **O** may move away the container **5** taking it to the medical waste disposal department.

Therefore, the steps **i)** of the recovery and **j)** of collection of the dirty water may be autonomously carried out by the collaborative robot **3** and/or by the autonomous robot **4.**

Also the materials contained in the medical kit **6** and used during the treatment may be moved away by the autonomous robot **4** or by a healthcare operator **O,** therefore reducing the risk of infection.

As described above, should there arise the need for a new medical kit **6,** the autonomous robot **4** may be instructed to retrieve another one from the closest buffer **8.**

During the treatment, the audio **30** and LED lights **31** system allows the collaborative robot **3** to carry out musicotherapy and/or chromotherapy upon request.

Suitably, on the articulated arm **12,** the collaborative robot **3** has a position sensor **48** preferably near the dispenser **13.**

This position sensor **48** is adapted to monitor the points of the patient **P** where the dispensing and distribution of the clean and decontaminated water occurs, verifying whether these points are the same points indicated in the protocol of the implemented smart contract.

In order to avoid cross contamination from one area to another on the body of the patient **P,** the collaborative robot **3** may also use the video cameras **32** to follow the gestures of the healthcare operators **O** to detect the parts of the body of the patient **P** where the operator has placed the hands and whether the area corresponds with the area set in the smart contract.

In case of errors, for example should the operators **O** have also touched the area of the trunk of the patient **P** while instead the smart contract indicates the head alone as the area to be treated, the collaborative robot **3** may promptly warn the healthcare operators **O** of the erroneous action in progress.

The collaborative robot **3** warns the healthcare operators **O** even in case of incongruence of the parameters with respect to the smart contract, for example a different ozone concentration in the water with respect to the predetermined one, a time duration of the treatment different from the predetermined one, etc.

Therefore, the collaborative robot **3** is configured to provide to at least one healthcare operator **O** information for supporting the performance of each protocol.

Suitably, the collaborative robot **3** is configured to collect data relating to the treatment of each individual patient **P** such as the date and duration of the treatment, the geolocation of said patient **P,** the amount of water, the ozone concentration, the water temperature, the sequence of the disinfection steps and the materials used, and other useful information.

Thanks to the various data collection sensors and the constant verification of the smart contract, the collaborative robot **3** allows an appropriate and precise treatment of the patient **P.**

Advantageously, the collected data are transmitted by the collaborative robot 3 to the storage unit of the central server **7** and they are processed so as to make them available to the healthcare operators **O,** so as to issue documentation, evaluate the quality of the treatment with respect to standard parameters defined in the disinfection protocol carried out, track the use of the materials and provide improvements for future treatments.

As a matter of fact, the collected data are compared with other data available in literature (for example from previous treatments and/or from medical texts) and with data collected during previous treatments so as to verify whether improvements can be implemented and therefore evaluate the treatment carried out.

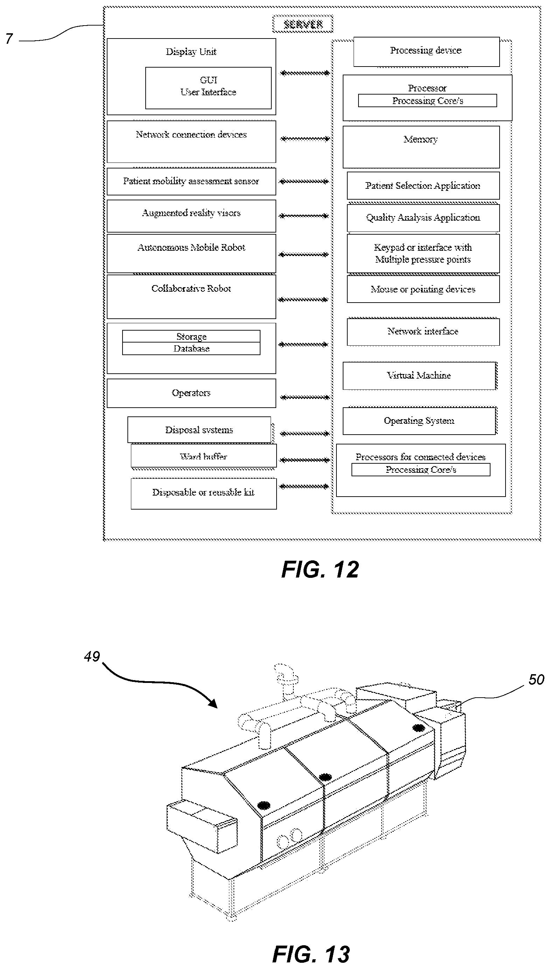

**FIG. 12** shows a diagram of how a central server **7** can be structured and how it can operate.

Suitably, even possible comments and/or information provided by the operators **O** may be stored, viewed and/or processed in the central server **7.**

For example, the healthcare operators **O** may provide information relating to condition of the patient **P,** the well-being thereof or the presence of lesions or other physical damage/ailments.

Advantageously, the central server **7** is configured to monitor and process the data and information of the automated disinfection treatments for the patients **P,** the status of the beds **2,** of the medical kits **6,** of the collaborative robots **3,** of the autonomous robots **4** and of the buffers **8** through the internet of things.

All this is possible since the beds **2,** the medical kits **6,** the collaborative robots **3,** the autonomous robots **4,** the central server **7** and the buffers **8** are operatively connected to each other and integrated to allow the controlled implementation of the standardised disinfection protocols as well as the exchange and storage of data and information relating to the disinfection treatments for the healthcare management and for the healthcare operators **O.**

At the end of each washing and disinfection treatment, the collaborative robot **3** is suitably disinfected using the second pump **14** to suction the liquid disinfectant, of a mixture of detergents through the disinfecting system **20** so as to disinfect all internal components.

Furthermore, the medical kits **6** used will be treated in a composting station **49,** like the one shown in **FIG. 13****,** having an autoclave therein.

Advantageously, each medical kit **6** may be made of compostable material, such as for example polylactic acid (PLA), cellulose, bioplastic or other derivatives of plant origin.

Therefore, treating the medical kits **6** in a composting station **49** will allow to obtain the compost, which may be used as a soil conditioner, intended to be used for farming.

As a matter of fact, the use thereof together with other nutritional organic substances improves the soil structure and the availability of nutritional elements which promote the growth of plants and vegetables.

Suitably, the composting station **49** may be provided with RFID readers **50** for precisely recognising which medical kits **6** are being disposed of to conclude the monitoring of the life cycle of the kit **6.**

In the light of the above, it is apparent that the method according to the invention achieves the pre-established objects and in particular it allows to drastically reduce the risk of contamination arising from pathogenic agents when treating the patients.

## Claims

1. An integrated non-therapeutic method for standardised and automated disinfection of patients (**P**) in hospital and/or home environments, comprising the following steps:
**a)** providing at least one bed (**2**) for each individual patient (**P**);
**b)** identifying a non-independent patient (**P**) to be washed and sanitised;
**characterized in that** it further comprises the following steps:
**c)** providing at least one central server (**7**) having a storage unit;
**d)** providing a plurality of standardised disinfection protocols with which there are associated digitalised instructions with ozonisation percentage, temperature and application time for washing and sanitising the patient (**P**) and storage thereof on said storage unit;
**e)** providing at least one collaborative robot (**3**) and at least one autonomous robot (**4**) connected to said at least one server (**7**) and having control means configured to allow the autonomous mobility in said hospital and/or home environments;
**f)** selecting from said storage unit a predetermined standardised disinfection protocol suitable for the identified patient (**P**) and allocating to said at least one collaborative robot (**3**) and to said autonomous robot (**4**) said selected protocol and the position of the bed of the identified patient (**P**);
**g)** collecting and decontaminating washing water through the pre-established ozonisation to confer to the water suitable bactericidal/antiviral properties;
**h)** disinfecting the identified patient (**P**) by distributing decontaminated and ozonised washing water on the skin of the patient (**P**) depending on the selected protocol;
**i)** recovering the dirty water coming from the washing and disinfection of each patient (**P**);
**j)** collecting in a special container (**5**) the contaminated washing water to be conveyed to the draining system;
wherein said step **g)** of collecting and decontaminating the water for washing each identified patient (**P**) is carried out autonomously by said collaborative robot (**3**) by means of a quick coupling pipe (**22**) connected to the water mains, an automatic ozone generation system (**16**), an ozone sensor (**17**), a first pump (**11**) and a clean water tank (**10**);
wherein said step **h)** of disinfecting the identified patient (**P**) is carried out autonomously by said collaborative robot (**3**) by approaching the bed (**2**) of the identified patient (**P**), collecting the decontaminated clean water from said tank (**10**) using said first pump (**11**), conveying the clean water to a dispenser (**13**) arranged at the end of a motor-driven articulated arm (**12**);
wherein said steps **i)** of recovering and **j)** of collecting the dirty water are carried out autonomously by said collaborative robot (**3**) and/or by said autonomous robot (**4**) by suctioning the dirty water from a collection compartment (**45**) positioned on the bed of the patient (**P**) using a second pump (**14**) and pouring thereof into said specific container (**5**);
wherein said collaborative robot (**3**) is configured to collect data relating to the treatment of each individual patient (**P**);
wherein said data are transmitted by said collaborative robot (**3**) to a storage unit connected to at least one central server (**7**) and they are processed to make them available to the healthcare operators (**O**).

2. Method as claimed in claim 1, wherein the data relating to the treatment of each individual patient (**P**) collected by said collaborative robot (**3**) relate to the date and duration of the treatment, the geolocation of the patient (**P**), the amount of water, the ozone concentration, the water temperature, the sequence of the disinfection steps and the materials used.

3. Method as claimed in claim 1, wherein each digitalised standardised disinfection protocol is encoded in a respective smart contract stored in said storage unit.

4. Method as claimed in claim 3, wherein the information contained in each specific smart contract are sent by said at least one central server (**7**) to said at least one collaborative robot (**3**) and/or to said autonomous robot (**4**) to autonomously carry out said steps **g)** to **j).**

5. Method as claimed in claim 1, wherein said collaborative robot (**3**) is configured to provide to at least one healthcare operator (**O**) instructions for supporting the performance of each selected protocol.

6. Method as claimed in claim 1, wherein said at least one autonomous robot (**4**) is configured to supply clean water to said at least one collaborative robot (**3**) to fill said at least one tank (**10**).

7. Method as claimed in claim 1, wherein said protocol provides for the use of medical kits (**6**) to carry out said step **h**) of disinfecting patients (**P**) under safe health conditions.

8. Method as claimed in claim 7, wherein the materials of said medical kit (**6**) used during the treatment are removable by the autonomous robot (**4**) or by at least one healthcare operator (**O**) to reduce the risk of infection.

9. Method as claimed in claim 1, wherein each healthcare operator (**O**) is equipped with smart-glasses (**47**) which are connected to said collaborative robot (**3**) and to said central server (**7**), through said smart-glasses (**47**) said collaborative robot (**3**) is adapted to display a pressure ulcer at a given stage and size, and to select a protocol indicated by the structure for pressure ulcers with similar characteristics.

10. Method as claimed in claim 9, wherein the healthcare operators (**O**) may photograph the ulcer through a simple voice command, the images thus acquired being sent to the central server (**7**) so as to be stored and they may be subsequently analysed or used as comparison with others so as to follow and evaluate the condition of the lesion/s so as to implement the best possible procedures.

11. A system (**1**) for carrying out the standardised and automated disinfection of patients (**P**) in hospital and/or home environments according to one or more of the preceding claims, comprising:
- at least one bed (**2**) for a bedridden patient (**P**) and means for identifying a patient (**P**) to be washed and sanitised;
**characterized in that** it further comprises:
- at least one central server (**7**) with a storage unit on which there are stored disinfection protocols which are standardised and digitalised in the form of smart contracts;
- at least one collaborative robot (**3**);
- at least one autonomous robot (**4**);
- at least one medical kit (**6**);
- at least one buffer (**8**) for collecting and distributing medical kits (**6**);
wherein said at least one collaborative robot (**3**) and said at least one autonomous robot (**4**) comprise means for collecting and transmitting data relating to the treatment of the identified patient (**P**), collecting and decontaminating washing water, disinfecting the identified patient (**P**) by distributing decontaminated and ozonised washing water on the skin of the patient (**P**), recovering the dirty water coming from the washing and disinfection of each patient (**P**);
- means for recovering and collecting in special containers (**5**) the dirty water coming from the washing and disinfection of each patient (**P**) to be conveyed to the draining system;
wherein said at least one collaborative robot (**3**) comprises a tank (**10**) for clean water, a first pump (**11**) for dispensing clean water, an articulated arm (**12**) provided with dispenser (**13**), an ozone generation system (**16**), an ozone sensor (**17**), a thermostat (**19**), a second pump (**14**) for suctioning dirty water;
wherein said at least one autonomous robot (**4**), said at least one central server (**7**) and said at least one buffer (**8**) are operatively connected and integrated to allow the controlled implementation of said standardised disinfection protocols as well as the exchange and storage of data and information relating to disinfection treatments for the healthcare management and for one or more healthcare operators (**O**);
wherein said at least one server (**7**) is configured to monitor and process said data and said information, the condition of said beds (**2**) for bedridden patients (**P**), of the medical kits (**6**), of said at least one collaborative robot (**3**) and of said at least one autonomous robot (**4**) through internet of things.

12. System as claimed in claim 11, wherein each bed (**2**) for each patient (**P**) is provided with a pressure sensor (**9**) for detecting the presence and mobility of the patient (**P**), and with a unique RFID tag.

13. System as claimed in claim 11, wherein said at least one medical kit (**6**) comprises disposable or reusable medical devices and a flexible laminar sheet (**43**), each kit (**6**) being associated with a unique RFID tag, said flexible laminar sheet (**43**) being provided with hooking means (**44**) for the bed (**2**) and with a plurality of compartments (**45**) intended, as a whole, to house a patient (**P**).

14. System as claimed in claim 11, wherein said at least one collaborative robot (**3**) further comprises a disinfecting system (**20**) and a pair of touchscreen monitors (**21,21**'), a control unit (**25**), drive means (**26**), a plurality of movement and geolocation sensors (**27**), an RFID reader (**28**), a microphone (**29**), an audio system (**30**), LED lights (**31**), a plurality of video cameras (**32**), as well as self-diagnosis means (**33**).

15. System as claimed in claim 11, wherein said at least one autonomous robot (**4**) comprises a plurality of internal compartments (**35**), a pair of touchscreen monitors (**36, 36'**), an RFID reader (**37**), a control unit, drive means (**38**) and a plurality of movement and geolocation sensors (**39**).

16. System as claimed in claim 11, wherein said at least one buffer (**8**) comprises a plurality of internal compartments (**40**), for the safe collection and controlled distribution of said medical kits (**6**), a touchscreen monitor (**41**), an RFID reader (**42**), a control unit and a recognition system adapted to interact with the operators (**O**) and with said at least one autonomous robot (**4**).

17. System as claimed in claim 11, wherein smart-glasses (**47**) are provided which can be worn by each healthcare operator (**O**) and connected to at least said collaborative robot (**3**) and/or to said at least one server (**7**), said smart-glasses (**47**) having lenses with an integrated micro-screen for providing the same information available on said touchscreen monitor (**21, 21'**) of said at least one collaborative robot (**3**) and for allowing each healthcare operator (**O**) to operate continuously and without looking away from the patient (**P**), said micro-screens integrated in the lenses of said smart-glasses (**47**) being configured to allow the healthcare operators (**O**) to simultaneously monitor the point featuring the lesion and the information sent by the collaborative robot (**3**).

## Patentansprüche

1. Ein integriertes nicht-therapeutisches Verfahren zur standardisierten und automatisierten Desinfektion von Patienten (P) in Krankenhaus- und/oder häuslichen Umgebungen, umfassend die folgenden Schritte:
a) Bereitstellen mindestens eines Bettes (2) für jeden einzelnen Patienten (P);
b) Identifizieren eines nicht selbständigen Patienten (P), der gewaschen und desinfiziert werden soll;
**dadurch gekennzeichnet, dass** es ferner die folgenden Schritte umfasst:
c) Bereitstellen mindestens eines zentralen Servers (7) mit einer Speichereinheit;
d) Bereitstellen einer Vielzahl standardisierter Desinfektionsprotokolle, denen digitalisierte Anweisungen mit Ozonisierungsprozentsatz, Temperatur und Anwendungszeit zum Waschen und Desinfizieren des Patienten (P) zugeordnet sind, sowie Speichern derselben auf der genannten Speichereinheit;
e) Bereitstellen mindestens eines kollaborativen Roboters (3) und mindestens eines autonomen Roboters (4), die mit dem mindestens einen Server (7) verbunden sind und Steuerungsmittel aufweisen, die so konfiguriert sind, dass sie die autonome Mobilität in den genannten Krankenhaus- und/oder häuslichen Umgebungen ermöglichen;
f) Auswählen aus der genannten Speichereinheit eines vorbestimmten standardisierten Desinfektionsprotokolls, das für den identifizierten Patienten (P) geeignet ist, und Zuweisen des ausgewählten Protokolls sowie der Position des Bettes des identifizierten Patienten (P) an den mindestens einen kollaborativen Roboter (3) und den autonomen Roboter (4);
g) Sammeln und Dekontaminieren von Waschwasser mittels der vorgegebenen Ozonisierung, um dem Wasser geeignete bakterizide/antivirale Eigenschaften zu verleihen;
h) Desinfizieren des identifizierten Patienten (P) durch Verteilen von dekontaminiertem und ozonisiertem Waschwasser auf der Haut des Patienten (P) in Abhängigkeit von dem ausgewählten Protokoll;
i) Rückgewinnen des verschmutzten Wassers, das aus dem Waschen und der Desinfektion jedes Patienten (P) stammt;
j) Sammeln des kontaminierten Waschwassers in einem speziellen Behälter (5), der dem Entwässerungssystem zugeführt wird;
wobei der genannte Schritt g) des Sammelns und Dekontaminierens des Wassers zum Waschen jedes identifizierten Patienten (P) autonom durch den genannten kollaborativen Roboter (3) mittels einer Schnellkupplungsleitung (22), die mit dem Wassernetz verbunden ist, eines automatischen Ozonerzeugungssystems (16), eines Ozonsensors (17), einer ersten Pumpe (11) und eines Reinwassertanks (10) durchgeführt wird;
wobei der genannte Schritt h) des Desinfizierens des identifizierten Patienten (P) autonom durch den genannten kollaborativen Roboter (3) durchgeführt wird, indem sich dieser dem Bett (2) des identifizierten Patienten (P) nähert, das dekontaminierte Reinwasser mittels der genannten ersten Pumpe (11) aus dem genannten Tank (10) entnimmt und das Reinwasser zu einem am Ende eines motorgetriebenen Gelenkarms (12) angeordneten Spender (13) fördert;
wobei die genannten Schritte i) des Rückgewinnens und j) des Sammelns des verschmutzten Wassers autonom durch den genannten kollaborativen Roboter (3) und/oder durch den genannten autonomen Roboter (4) durchgeführt werden, indem das verschmutzte Wasser mittels einer zweiten Pumpe (14) aus einem auf dem Bett des Patienten (P) angeordneten Sammelraum (45) abgesaugt und in den genannten speziellen Behälter (5) eingeleitet wird;
wobei der genannte kollaborative Roboter (3) dazu konfiguriert ist, Daten bezüglich der Behandlung jedes einzelnen Patienten (P) zu erfassen;
wobei die genannten Daten von dem genannten kollaborativen Roboter (3) an eine mit mindestens einem zentralen Server (7) verbundene Speichereinheit übertragen und verarbeitet werden, um sie den Gesundheitsfachkräften (O) verfügbar zu machen.

2. Verfahren nach Anspruch 1, wobei die von dem genannten kollaborativen Roboter (3) erfassten Daten bezüglich der Behandlung jedes einzelnen Patienten (P) das Datum und die Dauer der Behandlung, die Geolokalisierung des Patienten (P), die Wassermenge, die Ozonkonzentration, die Wassertemperatur, die Reihenfolge der Desinfektionsschritte und die verwendeten Materialien betreffen.

3. Verfahren nach Anspruch 1, wobei jedes digitalisierte standardisierte Desinfektionsprotokoll in einem jeweiligen Smart Contract codiert ist, der in der genannten Speichereinheit gespeichert ist.

4. Verfahren nach Anspruch 3, wobei die in jedem spezifischen Smart Contract enthaltenen Informationen von dem mindestens einen zentralen Server (7) an den mindestens einen kollaborativen Roboter (3) und/oder an den autonomen Roboter (4) gesendet werden, um die genannten Schritte g) bis j) autonom auszuführen.

5. Verfahren nach Anspruch 1, wobei der genannte kollaborative Roboter (3) dazu konfiguriert ist, mindestens einer Gesundheitsfachkraft (O) Anweisungen zur Unterstützung der Durchführung jedes ausgewählten Protokolls bereitzustellen.

6. Verfahren nach Anspruch 1, wobei der mindestens eine autonome Roboter (4) dazu konfiguriert ist, den mindestens einen kollaborativen Roboter (3) mit Reinwasser zu versorgen, um den mindestens einen Tank (10) zu befüllen.

7. Verfahren nach Anspruch 1, wobei das genannte Protokoll die Verwendung medizinischer Kits (6) vorsieht, um den genannten Schritt h) des Desinfizierens von Patienten (P) unter sicheren gesundheitlichen Bedingungen durchzuführen.

8. Verfahren nach Anspruch 7, wobei die während der Behandlung verwendeten Materialien des genannten medizinischen Kits (6) durch den autonomen Roboter (4) oder durch mindestens eine Gesundheitsfachkraft (O) entfernt werden können, um das Infektionsrisiko zu verringern.

9. Verfahren nach Anspruch 1, wobei jede Gesundheitsfachkraft (O) mit einer Smart-Brille (47) ausgestattet ist, die mit dem genannten kollaborativen Roboter (3) und dem genannten zentralen Server (7) verbunden ist, wobei der genannte kollaborative Roboter (3) über die genannte Smart-Brille (47) dazu eingerichtet ist, ein Druckgeschwür in einem gegebenen Stadium und einer gegebenen Größe anzuzeigen und ein von der Einrichtung angegebenes Protokoll für Druckgeschwüre mit ähnlichen Merkmalen auszuwählen.

10. Verfahren nach Anspruch 9, wobei die Gesundheitsfachkräfte (O) das Geschwür mittels eines einfachen Sprachbefehls fotografieren können, wobei die auf diese Weise erfassten Bilder an den zentralen Server (7) gesendet werden, damit sie gespeichert werden und anschließend analysiert oder zum Vergleich mit anderen verwendet werden können, um den Zustand der Läsion(en) zu verfolgen und zu bewerten und dadurch die bestmöglichen Verfahren umzusetzen.

11. System (1) zur Durchführung der standardisierten und automatisierten Desinfektion von Patienten (P) in Krankenhaus- und/oder häuslichen Umgebungen gemäß einem oder mehreren der vorhergehenden Ansprüche, umfassend:
- mindestens ein Bett (2) für einen bettlägerigen Patienten (P) und Mittel zum Identifizieren eines Patienten (P), der gewaschen und desinfiziert werden soll;
**dadurch gekennzeichnet, dass** es ferner umfasst:
- mindestens einen zentralen Server (7) mit einer Speichereinheit, auf welcher standardisierte und in Form von Smart Contracts digitalisierte Desinfektionsprotokolle gespeichert sind;
- mindestens einen kollaborativen Roboter (3);
- mindestens einen autonomen Roboter (4);
- mindestens ein medizinisches Kit (6);
- mindestens einen Puffer (8) zum Sammeln und Verteilen medizinischer Kits (6);
wobei der mindestens eine kollaborative Roboter (3) und der mindestens eine autonome Roboter (4) Mittel zum Erfassen und Übertragen von Daten bezüglich der Behandlung des identifizierten Patienten (P), zum Sammeln und Dekontaminieren von Waschwasser, zum Desinfizieren des identifizierten Patienten (P) durch Verteilen von dekontaminiertem und ozonisiertem Waschwasser auf der Haut des Patienten (P) sowie zum Rückgewinnen des verschmutzten Wassers, das aus dem Waschen und der Desinfektion jedes Patienten (P) stammt, umfassen;
- Mittel zum Rückgewinnen und Sammeln des verschmutzten Wassers, das aus dem Waschen und der Desinfektion jedes Patienten (P) stammt, in speziellen Behältern (5), die dem Entwässerungssystem zugeführt werden;
wobei der mindestens eine kollaborative Roboter (3) einen Tank (10) für Reinwasser, eine erste Pumpe (11) zur Ausgabe von Reinwasser, einen mit einem Spender (13) versehenen Gelenkarm (12), ein Ozonerzeugungssystem (16), einen Ozonsensor (17), einen Thermostaten (19) sowie eine zweite Pumpe (14) zum Absaugen von verschmutztem Wasser umfasst;
wobei der mindestens eine autonome Roboter (4), der mindestens eine zentrale Server (7) und der mindestens eine Puffer (8) betriebsmäßig verbunden und integriert sind, um die gesteuerte Umsetzung der genannten standardisierten Desinfektionsprotokolle sowie den Austausch und die Speicherung von Daten und Informationen bezüglich der Desinfektionsbehandlungen für das Gesundheitsmanagement und für eine oder mehrere Gesundheitsfachkräfte (O) zu ermöglichen;
wobei der mindestens eine Server (7) dazu konfiguriert ist, die genannten Daten und Informationen, den Zustand der genannten Betten (2) für bettlägerige Patienten (P), der medizinischen Kits (6), des mindestens einen kollaborativen Roboters (3) und des mindestens einen autonomen Roboters (4) mittels Internet der Dinge zu überwachen und zu verarbeiten.

12. System nach Anspruch 11, wobei jedes Bett (2) für jeden Patienten (P) mit einem Drucksensor (9) zum Erfassen der Anwesenheit und Mobilität des Patienten (P) und mit einem eindeutigen RFID-Tag versehen ist.

13. System nach Anspruch 11, wobei das mindestens eine medizinische Kit (6) Einweg- oder wiederverwendbare medizinische Vorrichtungen und eine flexible laminare Folie (43) umfasst, wobei jedes Kit (6) mit einem eindeutigen RFID-Tag verbunden ist und die genannte flexible laminare Folie (43) mit Befestigungsmitteln (44) für das Bett (2) und mit einer Vielzahl von Aufnahmebereichen (45) versehen ist, die insgesamt dazu bestimmt sind, einen Patienten (P) aufzunehmen.

14. System nach Anspruch 11, wobei der mindestens eine kollaborative Roboter (3) ferner ein Desinfektionssystem (20), ein Paar Touchscreen-Monitore (21, 21'), eine Steuereinheit (25), Antriebsmittel (26), eine Vielzahl von Bewegungs- und Geolokalisierungssensoren (27), ein RFID-Lesegerät (28), ein Mikrofon (29), ein Audiosystem (30), LED-Leuchten (31), eine Vielzahl von Videokameras (32) sowie Selbstdiagnosemittel (33) umfasst.

15. System nach Anspruch 11, wobei der mindestens eine autonome Roboter (4) eine Vielzahl von internen Fächern (35), ein Paar Touchscreen-Monitore (36, 36'), ein RFID-Lesegerät (37), eine Steuereinheit, Antriebsmittel (38) und eine Vielzahl von Bewegungs- und Geolokalisierungssensoren (39) umfasst.

16. System nach Anspruch 11, wobei der mindestens eine Puffer (8) eine Vielzahl von internen Fächern (40) zur sicheren Sammlung und kontrollierten Verteilung der genannten medizinischen Kits (6), einen Touchscreen-Monitor (41), ein RFID-Lesegerät (42), eine Steuereinheit und ein Erkennungssystem umfasst, das dazu eingerichtet ist, mit den Gesundheitsfachkräften (O) und mit dem mindestens einen autonomen Roboter (4) zu interagieren.

17. System nach Anspruch 11, wobei Smart-Brillen (47) vorgesehen sind, die von jeder Gesundheitsfachkraft (O) getragen werden können und mit zumindest dem genannten kollaborativen Roboter (3) und/oder mit dem mindestens einen Server (7) verbunden sind, wobei die genannten Smart-Brillen (47) Gläser mit einem integrierten Mikrobildschirm aufweisen, um dieselben Informationen bereitzustellen, die auf dem genannten Touchscreen-Monitor (21, 21') des mindestens einen kollaborativen Roboters (3) verfügbar sind, und um jeder Gesundheitsfachkraft (O) zu ermöglichen, kontinuierlich und ohne den Blick vom Patienten (P) abzuwenden zu arbeiten, wobei die in die Gläser der genannten Smart-Brillen (47) integrierten Mikrobildschirme dazu konfiguriert sind, den Gesundheitsfachkräften (O) zu ermöglichen, gleichzeitig die Stelle mit der Läsion und die von dem kollaborativen Roboter (3) gesendeten Informationen zu überwachen.

## Revendications

1. En procédé intégré non thérapeutique de désinfection standardisée et automatisée de patients (P) dans des environnements hospitaliers et/ou domestiques, comprenant les étapes suivantes:
a) fournir au moins un lit (2) pour chaque patient individuel (P);
b) identifier un patient non autonome (P) devant être lavé et désinfecté;
**caractérisé en ce qu'**il comprend en outre les étapes suivantes:
c) fournir au moins un serveur central (7) comportant une unité de stockage;
d) fournir une pluralité de protocoles de désinfection standardisés auxquels sont associées des instructions numérisées comportant un pourcentage d'ozonisation, une température et un temps d'application pour le lavage et la désinfection du patient (P), et stocker ceux-ci sur ladite unité de stockage;
e) fournir au moins un robot collaboratif (3) et au moins un robot autonome (4) connectés audit au moins un serveur (7) et comportant des moyens de commande configurés pour permettre la mobilité autonome dans lesdits environnements hospitaliers et/ou domestiques;
f) sélectionner, à partir de ladite unité de stockage, un protocole de désinfection standardisé prédéterminé adapté au patient (P) identifié et attribuer audit au moins un robot collaboratif (3) et audit robot autonome (4) ledit protocole sélectionné ainsi que la position du lit du patient (P) identifié;
g) collecter et décontaminer de l'eau de lavage au moyen de l'ozonisation prédéterminée afin de conférer à l'eau des propriétés bactéricides/antivirales appropriées;
h) désinfecter le patient (P) identifié en distribuant de l'eau de lavage décontaminée et ozonisée sur la peau du patient (P) en fonction du protocole sélectionné;
i) récupérer l'eau souillée provenant du lavage et de la désinfection de chaque patient (P);
j) collecter l'eau de lavage contaminée dans un récipient spécifique (5) destiné à être acheminé vers le système d'évacuation;
dans lequel ladite étape g) de collecte et de décontamination de l'eau destinée au lavage de chaque patient (P) identifié est réalisée de manière autonome par ledit robot collaboratif (3) au moyen d'une conduite à raccord rapide (22) reliée au réseau d'eau, d'un système automatique de génération d'ozone (16), d'un capteur d'ozone (17), d'une première pompe (11) et d'un réservoir d'eau propre (10);
dans lequel ladite étape h) de désinfection du patient (P) identifié est réalisée de manière autonome par ledit robot collaboratif (3) en s'approchant du lit (2) du patient (P) identifié, en prélevant l'eau propre décontaminée à partir dudit réservoir (10) au moyen de ladite première pompe (11), et en acheminant l'eau propre vers un distributeur (13) disposé à l'extrémité d'un bras articulé motorisé (12);
dans lequel lesdites étapes i) de récupération et j) de collecte de l'eau souillée sont réalisées de manière autonome par ledit robot collaboratif (3) et/ou par ledit robot autonome (4) en aspirant l'eau souillée à partir d'un compartiment de collecte (45) disposé sur le lit du patient (P) au moyen d'une seconde pompe (14) et en la déversant dans ledit récipient spécifique (5);
dans lequel ledit robot collaboratif (3) est configuré pour collecter des données relatives au traitement de chaque patient individuel (P);
dans lequel lesdites données sont transmises par ledit robot collaboratif (3) à une unité de stockage connectée à au moins un serveur central (7) et sont traitées afin d'être mises à la disposition des opérateurs de santé (O).

2. Procédé selon la revendication 1, dans lequel les données relatives au traitement de chaque patient individuel (P), collectées par ledit robot collaboratif (3), concernent la date et la durée du traitement, la géolocalisation du patient (P), la quantité d'eau, la concentration en ozone, la température de l'eau, la séquence des étapes de désinfection et les matériaux utilisés.

3. Procédé selon la revendication 1, dans lequel chaque protocole de désinfection standardisé numérisé est codé dans un smart contract respectif stocké dans ladite unité de stockage.

4. Procédé selon la revendication 3, dans lequel les informations contenues dans chaque smart contract spécifique sont envoyées par ledit au moins un serveur central (7) audit au moins un robot collaboratif (3) et/ou audit robot autonome (4) afin de réaliser de manière autonome lesdites étapes g) à j).

5. Procédé selon la revendication 1, dans lequel ledit robot collaboratif (3) est configuré pour fournir à au moins un opérateur de santé (O) des instructions destinées à assister l'exécution de chaque protocole sélectionné.

6. Procédé selon la revendication 1, dans lequel ledit au moins un robot autonome (4) est configuré pour fournir de l'eau propre audit au moins un robot collaboratif (3) afin de remplir ledit au moins un réservoir (10).

7. Procédé selon la revendication 1, dans lequel ledit protocole prévoit l'utilisation de kits médicaux (6) afin de réaliser ladite étape h) de désinfection des patients (P) dans des conditions sanitaires sûres.

8. Procédé selon la revendication 7, dans lequel les matériaux dudit kit médical (6) utilisés pendant le traitement peuvent être retirés par le robot autonome (4) ou par au moins un opérateur de santé (O) afin de réduire le risque d'infection.

9. Procédé selon la revendication 1, dans lequel chaque opérateur de santé (O) est équipé de lunettes intelligentes (47) qui sont connectées audit robot collaboratif (3) et audit serveur central (7), ledit robot collaboratif (3) étant adapté, par l'intermédiaire desdites lunettes intelligentes (47), à afficher une escarre à un stade et d'une taille donnés, et à sélectionner un protocole indiqué par l'établissement pour des escarres présentant des caractéristiques similaires.

10. Procédé selon la revendication 9, dans lequel les opérateurs de santé (O) peuvent photographier l'escarre au moyen d'une simple commande vocale, les images ainsi acquises étant envoyées au serveur central (7) afin d'y être stockées et pouvant ensuite être analysées ou utilisées à des fins de comparaison avec d'autres images afin de suivre et d'évaluer l'état de la ou des lésions, de manière à mettre en œuvre les meilleures procédures possibles.

11. Système (1) destiné à réaliser la désinfection standardisée et automatisée de patients (P) dans des environnements hospitaliers et/ou domestiques selon une ou plusieurs des revendications précédentes, comprenant:
- au moins un lit (2) pour un patient alité (P) et des moyens d'identification d'un patient (P) devant être lavé et désinfecté;
**caractérisé en ce qu'**il comprend en outre:
- au moins un serveur central (7) comportant une unité de stockage sur laquelle sont stockés des protocoles de désinfection standardisés et numérisés sous la forme de smart contracts;
- au moins un robot collaboratif (3);
- au moins un robot autonome (4);
- au moins un kit médical (6);
- au moins un tampon (8) destiné à la collecte et à la distribution de kits médicaux (6);
dans lequel ledit au moins un robot collaboratif (3) et ledit au moins un robot autonome (4) comprennent des moyens de collecte et de transmission de données relatives au traitement du patient (P) identifié, de collecte et de décontamination de l'eau de lavage, de désinfection du patient (P) identifié par distribution d'eau de lavage décontaminée et ozonisée sur la peau du patient (P), et de récupération de l'eau souillée provenant du lavage et de la désinfection de chaque patient (P);
- des moyens de récupération et de collecte, dans des récipients spécifiques (5), de l'eau souillée provenant du lavage et de la désinfection de chaque patient (P) destinée à être acheminée vers le système d'évacuation;
dans lequel ledit au moins un robot collaboratif (3) comprend un réservoir (10) pour l'eau propre, une première pompe (11) pour la distribution de l'eau propre, un bras articulé (12) muni d'un distributeur (13), un système de génération d'ozone (16), un capteur d'ozone (17), un thermostat (19) et une seconde pompe (14) destinée à l'aspiration de l'eau souillée;
dans lequel ledit au moins un robot autonome (4), ledit au moins un serveur central (7) et ledit au moins un tampon (8) sont reliés et intégrés de manière opérationnelle afin de permettre la mise en œuvre contrôlée desdits protocoles de désinfection standardisés ainsi que l'échange et le stockage de données et d'informations relatives aux traitements de désinfection pour la gestion des soins de santé et pour un ou plusieurs opérateurs de santé (O);
dans lequel ledit au moins un serveur (7) est configuré pour surveiller et traiter lesdites données et lesdites informations, l'état desdits lits (2) pour patients alités (P), des kits médicaux (6), dudit au moins un robot collaboratif (3) et dudit au moins un robot autonome (4) au moyen de l'internet des objets.

12. Système selon la revendication 11, dans lequel chaque lit (2) pour chaque patient (P) est muni d'un capteur de pression (9) destiné à détecter la présence et la mobilité du patient (P), et d'une étiquette RFID unique.

13. Système selon la revendication 11, dans lequel ledit au moins un kit médical (6) comprend des dispositifs médicaux jetables ou réutilisables et une feuille laminaire flexible (43), chaque kit (6) étant associé à une étiquette RFID unique, ladite feuille laminaire flexible (43) étant munie de moyens de fixation (44) pour le lit (2) et d'une pluralité de compartiments (45) destinés, dans leur ensemble, à recevoir un patient (P).

14. Système selon la revendication 11, dans lequel ledit au moins un robot collaboratif (3) comprend en outre un système de désinfection (20) et une paire de moniteurs à écran tactile (21, 21'), une unité de commande (25), des moyens d'entraînement (26), une pluralité de capteurs de mouvement et de géolocalisation (27), un lecteur RFID (28), un microphone (29), un système audio (30), des lumières à LED (31), une pluralité de caméras vidéo (32), ainsi que des moyens d'autodiagnostic (33).

15. Système selon la revendication 11, dans lequel ledit au moins un robot autonome (4) comprend une pluralité de compartiments internes (35), une paire de moniteurs à écran tactile (36, 36'), un lecteur RFID (37), une unité de commande, des moyens d'entraînement (38) et une pluralité de capteurs de mouvement et de géolocalisation (39).

16. Système selon la revendication 11, dans lequel ledit au moins un tampon (8) comprend une pluralité de compartiments internes (40), destinés à la collecte sécurisée et à la distribution contrôlée desdits kits médicaux (6), un moniteur à écran tactile (41), un lecteur RFID (42), une unité de commande et un système de reconnaissance adapté pour interagir avec les opérateurs de santé (O) et avec ledit au moins un robot autonome (4).

17. Système selon la revendication 11, dans lequel des lunettes intelligentes (47) sont prévues, pouvant être portées par chaque opérateur de santé (O) et connectées au moins audit robot collaboratif (3) et/ou audit au moins un serveur (7), lesdites lunettes intelligentes (47) comportant des verres munis d'un micro-écran intégré destiné à fournir les mêmes informations que celles disponibles sur ledit moniteur à écran tactile (21, 21') dudit au moins un robot collaboratif (3) et à permettre à chaque opérateur de santé (O) de travailler de manière continue sans détourner le regard du patient (P), lesdits micro-écrans intégrés dans les verres desdites lunettes intelligentes (47) étant configurés pour permettre aux opérateurs de santé (O) de surveiller simultanément la zone présentant la lésion et les informations envoyées par le robot collaboratif (3).
